(19) Europäisches Patentamt
European Patent Office
Office européen des brevets

(11) Veröffentlichungsnummer: **0 277 631 B1**

(12) **EUROPÄISCHE PATENTSCHRIFT**

(45) Veröffentlichungstag der Patentschrift: **30.10.91**

(51) Int. Cl.[5]: **C07D 215/18**, C07D 215/48

(21) Anmeldenummer: **88101453.4**

(22) Anmeldetag: **02.02.88**

# (54) Verfahren zur Herstellung von substituierten 3-Alkyl-chinolin-8-carbonsäuren.

(30) Priorität: **03.02.87 DE 3703113**

(43) Veröffentlichungstag der Anmeldung:
**10.08.88 Patentblatt 88/32**

(45) Bekanntmachung des Hinweises auf die Patenterteilung:
**30.10.91 Patentblatt 91/44**

(84) Benannte Vertragsstaaten:
**BE CH DE FR GB LI NL**

(56) Entgegenhaltungen:
**EP-A- 060 429**
**EP-A- 0 104 389**

**Organikum VEB Berlin,15.Aufl.1976,S.210-213; Houben-Weyl, Bd V/4,G.Thieme Verlag,1960,S.332-337**

**Chemical Abstracts, Band 100, Nr. 7, 13. Februar 1984, Columbus, Ohio, USA : A.I. Tochilkin & al. "Aromatic chlorination and iodination of 8-methylquino- line". Seite 567, Spalte 1, Zusammenfassung Nr. 51 427k and Khim. Geterotsikl. Soedin., (10), 1373-6 (1983)**

(73) Patentinhaber: **BASF Aktiengesellschaft**
**Carl-Bosch-Strasse 38**
**W-6700 Ludwigshafen(DE)**

(72) Erfinder: **Hagen, Helmut, Dr.**
**Max-Slevogt-Strasse 17 e**
**W-6710 Frankenthal(DE)**
Erfinder: **Kohler, Rolf-Dieter, Dr.**
**Amselweg 3**
**W-6803 Edingen-Neckarhausen(DE)**
Erfinder: **Dupuis, Jacques, Dr.**
**Bannwasserstrasse 37**
**W-6700 Ludwigshafen(DE)**

**EP 0 277 631 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von 8-Brommethyl-3-methyl-chinolinverbindungen der allgemeinen Formel (I)

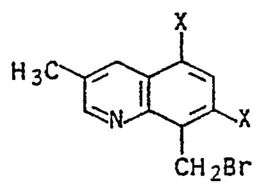

(I)

in der X mindestens ein Chloratom sowie gegebenenfalls Wasserstoff bedeutet, sowie die Verwendung der Chinolinverbindungen I zur Herstellung von Chinolin-8-carbonsäuren der Formel (II)

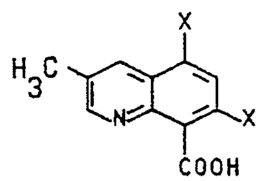

(II)

in der mindestens ein Substituent X Chlor und gegebenenfalls der andere Wasserstoff ist.

Chinolin-8-carbonsäuren II sind wertvolle Wirkstoffe auf dem Pflanzenschutzgebiet. Sie können durch Oxidation der entsprechenden 8-Brommethylchinolinverbindung mit Salpetersäure in Gegenwart von Schwefelsäure erhalten werden. Ein solches Verfahren ist aus der EP-A- 104 389 bekannt; es hat den Nachteil, nicht glatt zu verlaufen und liefert das gewünschte Zielprodukt nur mit geringer Ausbeute; außerdem sind die benötigten Zwischenprodukte der allgemeinen Formel (I) nur schwer in erforderlicher Reinheit erhältlich, weil die Bromierung nicht selektiv genug verläuft.

Der Erfindung lag daher die Aufgabe zugrunde, die erforderlichen 8-Brommethyl-3-methyl-chinolinverbindungen I in hoher Ausbeute und Reinheit bereitzustellen.

Demgemäß wurde ein Verfahren zur Herstellung von 8-Brommethyl-3-methyl-chinolinverbindungen der allgemeinen Formel (I)

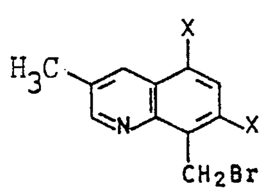

(I),

in der X mindestens ein Chloratom sowie gegebenenfalls Wasserstoff bedeutet, gefunden, das dadurch gekennzeichnet ist, daß man das entsprechende 3,8-Dimethylchinolin durch radikalische Bromierung mittels Brom bei Temperaturen von 0 bis 100 °C in einem mit Wasser nicht mischbaren, gegen Brom beständigen Lösungsmittel in Gegenwart von gegebenenfalls schwach sauer gepuffertem Wasser in die 8-Brommethylchinolinverbindung (I) überführt.

Als Lösungsmittel eignen sich - an sich bekannt - z.B. Nitrobenzol oder halogenierte Kohlenwasserstoffe wie Halogenbenzole, Chloroform oder Dichlorethan, d.h. mit Wasser nicht oder nicht unbegrenzt mischbare Lösungsmittel.

Die wäßrige Phase wird z.B. mit Alkaliacetat versetzt, wobei sich durch den freiwerdenden Bromwasserstoff eine Pufferwirkung im Bereich von pH 4 bid 5 ergibt. Insgesamt sollte die wäßrige Phase während der Umsetzung im wesentlichen pH 3 bis 6 aufweisen.

Die Brommethylverbindung erhält man durch Bromieren der entsprechenden Methylverbindung in Gegenwart eines Radikalbildners oder im Licht schon bei verhältnismäßig niedriger Temperatur; je nach Art des Radikalbildners bzw. je nach Lichtwellenlänge genügen schon 0 bis 20 °C; handelsübliche Radikalbildner erfordern u.U. bis zu 100 °C.

Als Radikalbildner verwendet man eines der handelsüblichen Mittel wie Azo-bis-isobutylronitril oder man

belichtet mit Strahlung einer ausreichend kurzen Wellenlänge, wie sie etwa das Entladungsspektrum einer Quecksilberdampflampe liefert.

Die Brommethylverbindung befindet sich nach der Umsetzung in der nichtwäßrigen Phase und kann unmittelbar mit wäßriger Schwefelsäure daraus extrahiert werden. Die Umsetzung kann verfahrenstechnisch mit der beabsichtigten nachfolgenden Oxidation in geeigneter Weise verbunden werden, wobei die Betriebsmittel wie Halogenkohlenwasserstoff und Schwefelsäure nach geeigneter Vorbehandlung wiederverwendet werden können.

Die zur Gewinnung von Chinolincarbonsäureverbindungen II nötige Oxidation der Brommethylchinolinverbindungen I geschieht zweckmäßig mit Salpetersäure in Gegenwart von Schwefelsäure, wobei sich gezeigt hat, daß auch dieser Teilschritt des Verfahrens für sich verbesserungsfähig ist. Vorteilhaft setzt man in die Brommethylverbindung (I) mit Salpetersäure in der Weise um, daß man ein Schwermetall zusetzt, das in Salpetersäure enthaltender, mäßig konzentrierter Schwefelsäure mehrwertige, mehrerer Wertigkeiten fähiger Ionen bildet.

Solche Schwermetalle liefernde Verbindungen sind z.B. Halogenide und Oxide von Vanadium, Chrom, Molybdän, Wolfram, Eisen, Kobalt, Cer, Nickel, Kupfer und gewisser Platinmetalle, z.B. Ruthenium oder Osmium. Der Einfachheit halber kann man die Oxide oder Halogenide, jedoch auch die Metalle selbst in elementarer Form zusetzen. Besonders wirksam und daher bevorzugt ist Mangandioxid.

Die Umsetzungsbedingungen können gegenüber dem bekannten Verfahren günstiger gestaltet werden; man arbeitet i.a. bei Temperaturen von 50 bis 100 °C, d.h. unterhalb des Siedepunkts des Systems, der bei atmosphärischem Druck in der Nähe von 140 °C liegt. Dabei liegt eine Lösung der Brommethylverbindung in etwa 50 bis 80 %iger Schwefelsäure vor und es wird mäßig konzentrierte oder konzentrierte Salpetersäure verwendet. Praktisch geht man z.B. so vor, daß man die Brommethylverbindung in der Schwefelsäure bei etwa 80 bis 100 °C vorlegt und dann allmählich mit etwa 2,5 bis 5 Aquivalenten Salpetersäure - bezogen auf 1 mol der Bromverbindung - versetzt. Die Umsetzung verläuft innerhalb von 5 bis 6 Stunden oder - je nach Temperatur - auch schneller oder langsamer.

Man verdünnt mit Wasser, bringt auf pH 2,5 bis 3,5 (wobei alle Chinolinverbindungen einschließlich eventueller Nebenprodukte ausfallen) und gewinnt die Carbonsäure nach Ausziehen mit Methanol, Isopropanol o.ä. als Rückstand, der durch Umkristallisieren wenn nötig gereinigt werden kann.

Die Umsetzung kann absatzweise z.B. im Rührkessel oder nach den üblichen Regeln der Verfahrenstechnik auch fortlaufend vorgenommen werden, wobei Rührkessel mit nachgeschaltetem Rohrreaktor, Rührkesselkaskaden oder Rohrreaktoren verwendbar sind.

Beispiel 1

Herstellung von 8-Brommethyl-7-chlor-3-methylchinolin

9,6 g 7-Chlor-3,8-dimethylchinolin werden in 850 g 1,2-Dichlorbenzol gelöst; 6,2 g Natriumacetat, gelöst in 150 ml Wasser und anschließend 8 g Brom werden hinzugegeben und bei 15 °C während 5 bis 6 Stunden mit einer Quecksilber-Niederdrucklampe mit 150 Watt Stromaufnahme belichtet. Die Ausbeute wird chromatographisch bestimmt und beträgt 80 % der berechneten, bezogen auf umgesetztes Dimethylchinolin. Die Selektivität der Reaktion beträgt 88 % bei einem Umsatz von 91 %. In Tabelle 1 ist das Ergebnis der Variation der Reaktionsbedingungen zusammengefaßt.

Tabelle 1: Variation der Reaktionsbedingungen bei der photochemischen Bromierung von 9,6 g 7-Chlor-3,8-dimethylchinolin

| Lösungsmittel Art | Lösungsmittel Menge [g] | $Br_2$ [g] | Temperatur [°C] | Ausbeute* [%] | Umsatz** [%] |
|---|---|---|---|---|---|
| Dichlorbenzol | 850 | 8 | 15 | 80 | 91 |
| Chlorbenzol | 720 | 8 | 15 | 79 | 90 |
| Nitrobenzol | 780 | 8 | 20 | 78 | 91 |
| Di-, Tri- und Tetrachlormethan | je 650 | je 8 | 20 | 80-76 | 90 |
| Trifluortrichlorethan | 880 | 8 | 20 | 83 ** | 92 |
| Dichlorbenzol | 40 | 8 | 25 | 61 | 85 |

Tabelle 1: Fortsetzung

| Lösungsmittel Art | Lösungsmittel Menge [g] | $Br_2$ [g] | Temperatur [°C] | Ausbeute* [%] | Umsatz** [%] |
|---|---|---|---|---|---|
| Dichlormethan | 35 | 8 | 25-30 | 63 | 86 |
| Dichlorbenzol | 60 | 8 | 70 | 55 | 81 |
| Dichlorbenzol | 40 | 8 | 35 | 60 | 87 |
| Dichlorbenzol | 40 | 7,2 | 40 | 63 | 78 |
| Dichlorbenzol | 40 | 6,4 | 40 | 62 | 74 |
| Dichlorbenzol | 40 | 4 | 40 | 45 | 45 |

* bezogen auf eingesetztes 7-Chlor-3,8-dimethylchinolin

** nach einer Reaktionszeit von 10 Stunden

Beispiel 2

9,6 g 7-Chlor-3,8-dimethylchinolin werden in 65 g 1,2-Dichlorbenzol vorgelegt und 6,2 g Natriumacetat gelöst in 15 ml Wasser hinzugegeben. Nachdem auf 90°C erwärmt wurde, tropft man bei dieser Temperatur gleichzeitig eine Lösung von 0,07 g $\alpha,\alpha'$-Azoisobutyronitril in 4 g 1,2-Dichlorbenzol und eine Lösung von 8 g Brom in 4 g 1,2-Dichlorbenzol innerhalb von 10 min zu und rührt 1 Stunde nach. Ausbeute: 63 % (chromatographisch) , Umsatz: 87 %. Tabelle 2 zeigt wie die Ausbeute im verlauf der Reaktion sich ändert.

Tabelle 2

| Ausbeute in Abhängigkeit vom Umsatz | | | |
|---|---|---|---|
| Umsatz [%] | Brom [Äqu.] | Ausbeute [%], bezogen auf | |
| | | Eingesetztes Chinolin | Umgesetztes Chinolin |
| 9 | 0,2 | 8 | 89 |
| 23 | 0,9 | 19 | 83 |
| 44 | 0,5 | 35 | 80 |
| 70 | 0,7 | 53 | 76 |
| 76 | 0,8 | 58 | 76 |
| 85 | 1,0 | 62 | 73 |
| 97 | 1,4 | 67 | 70 |

Herstellung von 7-Chlor-3-methylchinolin-8-carbonsäure

270,5 g 8-Brommethyl-7-chlor-3-methylchinolin werden in 950 g 70 %iger Schwefelsäure gelöst, auf 90 °C erwärmt und 4,5 g Mangandioxid zugegeben. Anschließend werden 290 g 65 %ige Salpetersäure innerhalb von 5 Stunden zugetropft. Man läßt noch 2 Stunden stehen, kühlt ab und verdünnt mit Wasser auf das Doppelte.

Ein pH-Wert von 3 wird mit konzentrierter Natronlauge eingestellt und der Niederschlag abfiltriert.

Der feuchte Filterkuchen wird mit der gleichen Menge Methanol oder Isopropylalkohol aufgekocht und bei 40 °C filtriert. Die so erhaltene Säure hat eine Reinheit von 95 %. Die Ausbeute beträgt 96 %. Je nach Reinheitsanforderung kann mit verdünnter Natronlauge umgefällt werden (Reinheit: 98 %; Ausbeute: 92 %).

Beispiel 3

7-Chlor-3-methyl-chinolin-8-carbonsäure; Gesamtverfahren

191,5 g 7-Chlor-3,8-dimethylchinolin werden in 1300 g 1,2-Dichlorbenzol gelöst. 86,1 g Natriumacetat gelöst in 200 ml Wasser werden hinzugegeben. Es wird auf 90 °C erhitzt und 1,4 g $\alpha,\alpha'$-Azoisobutyronitril gelöst in 80 g 1,2-Dichlorobenzol einerseits und 112 g Brom, gelöst in 80 g 1,2-Dichlorbenzol andererseits bei dieser Temperatur innerhalb von 10 bis 20 min zugetropft. Nach 1 Stunde bei 90 °C wird die wäßrige Phase abgetrennt und die nichtwäßrige Phase bei dieser Temperatur einmal mit 750 g und einmal mit 250 g 70 %iger Schwefelsäure extrahiert.

Die Extrakte werden vereinigt und nach Zugabe von 4,5 Mangandioxid auf 90 bis 100 °C erwärmt. Eine Lösung aus 236 g 55 %iger Salpetersäure und 84 g konzentrierter Schwefelsäure wird dann innerhalb von 6 Stunden hinzugetropft. Nach 2 Stunden bei 90 °C wird das Gemisch abgekühlt, mit 2000 ml Wasser verdünnt und mit konzentrierter Natronlauge auf pH 3 gebracht. Der Niederschlag wird zweimal mit der gleichen Menge Methanol oder Isopropylalkohol aufgekocht und bei 60 °C abfiltriert. Die als Rückstand erhaltene 7-Chlor-3-methylchinolin-8-carbonsäure wird mit einer Ausbeute von 85 %. bezogen auf die umgesetzte Dimethylverbindung und mit einer Reinheit von 96 % isoliert (Schmp.: 239 bis 242 °C).

Man kann aus verdünnter Natronlauge umfällen und erhält dann 98 % reine Säure (Schmp.: 243 bis 244 °C) in einer Ausbeute von 82 %. Aus dem gesammelten Extrakt läßt sich Methanol abdestillieren und 74 g 7-Chlor-3,8-dimethylchinolin zurückgewinnen.

**Patentansprüche**

**1.** Verfahren zur Herstellung von 8-Brommethyl-3-methyl-chinolinverbindungen der allgemeinen Formel (I)

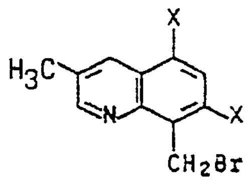

(I)

in der X mindestens ein Chloratom sowie gegebenenfalls Wasserstoff bedeutet, dadurch gekennzeichnet, daß man das entsprechende 3,8-Dimethylchinolin durch radikalische Bromierung mittels Brom bei Temperaturen von 0 bis 100 °C in einem mit Wasser nicht mischbaren, gegen Brom beständigen Lösungsmittel in Gegenwart von gegebenenfalls schwach sauer gepuffertem Wasser in die 8-Brommethyl-chinolinverbindung (I) überführt.

2. Verfahren nach Anspruch 1, dadurch gekennzeichnet, daß man als Puffer ein schwach sauer pufferndes System verwendet.

3. Verfahren nach Anspruch 1, dadurch gekennzeichnet, daß man ein Alkaliacetat verwendet.

4. Verfahren nach Anspruch 1, dadurch gekennzeichnet. daß man in an sich bekannter Weise die Umsetzung mit Hilfe eines Radikalbildners oder von Licht fördert.

5. Verwendung der nach einem der Ansprüche 1 bis 4 erhaltenen 8-Brommethyl-3-methylchinolinverbindung zur Herstellung der entsprechenden Chinolin-8-carbonsäure durch Oxidation mit Salpetersaure.

## Claims

1. A process for the preparation of an 8-bromo-methyl-3-methylquinoline compound of the formula (I)

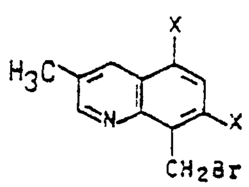

where X is at least one chlorine atom and possibly hydrogen, which comprises converting the corresponding 3,8-dimethylquinoline into the 8-bromomethylquinoline compound (I) by free-radical bromination using bromine at from 0 to 100 °C in a water-immiscible, bromine-stable solvent in the presence of water, unbuffered or buffered at a weakly acidic pH.

2. A process as claimed in claim 1, wherein the buffer used is a system which buffers at weakly acidic pH.

3. A process as claimed in claim 1, wherein an alkali metal acetate is used.

4. A process as claimed in claim 1, wherein the reaction is promoted in a conventional manner using a free radical initiator or light.

5. Use of an 8-bromomethyl-3-methylquinoline compound obtained as claimed in claim 1 or 2 or 3 or 4 for the preparation of the corresponding quinoline-8-carboxylic acid by oxidation with nitric acid.

## Revendications

1. Procédé de préparation de dérivés de 8-bromométhyl-3-méthyl-quinoléine de formule générale (I)

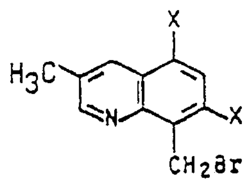

(I)

dans laquelle x représente au moins un atome de chlore et éventuellement de l'hydrogène, caractérisé par le fait que l'on transforme la 3,8-diméthylquinoléine correspondante, par bromation radicalaire au moyen de brome, à des températures comprises entre 0 et 100 degrés C, dans un solvant non miscible avec l'eau, stable au brome, en présence d'eau éventuellement tamponée avec un acide faible, en dérivé de 8-bromothyl-quinoléine (I).

2. Procédé selon la revendication 1, caractérisé par le fait que l'on utilise, comme tampon, un système à effet tamponnant faiblement acide.

3. Procédé selon la revendication 1, caractérisé par le fait que l'on utilise un acétate alcalin.

4. Procédé selon la revendication 1, caractérisé par le fait que l'on favorise la réaction par un produit engendrant des radicaux ou par de la lumière.

5. Utilisation du dérivé de 8-bromométhyl-3-méthylquinoléine obtenu selon l'une des revendications 1 à 4, pour préparer l'acide quinoléine-8-carboxylique correspondant par oxydation avec de l'acide nitrique.